# EUROPEAN PATENT SPECIFICATION

(11) **EP 3 991 676 B1**
(45) Date of publication and mention of the grant of the patent: **25.03.2026**
(21) Application number: 20905754.6
(22) Date of filing: 29.09.2020
(51) Int. Cl.: A61B 18/12, A61B 18/14, A61B 5/00, A61B 5/021

(54) **NERVE DETECTION DEVICE FOR AN ABLATION SYSTEM**
NERVENDETEKTIONSVORRICHTUNG FÜR EIN ABLATIONSSYSTEM
DISPOSITIF DE DÉTECTION DE NERFS POUR UN SYSTÈME D'ABLATION

(30) Priority: 26.12.2019 CN 201911368162; 13.02.2020 CN 202010090923
(43) Date of publication of application: 04.05.2022
(73) Proprietor: Shanghai Microport Ep Medtech Co., Ltd., Shanghai 201318 (CN)
(72) Inventor: YIN, Yuehui, Chongqing 402260 (CN); SUN, Yiyong, Shanghai 201318 (CN); SHEN, Liuping, Shanghai 201318 (CN); PENG, Yahui, Shanghai 201318 (CN); YU, Zhili, Shanghai 201318 (CN); ZHANG, Qingchun, Shanghai 201318 (CN)
(74) Representative: Patentship Patentanwaltsgesellschaft mbH
(86) International application number: PCT/CN2020/118973
(87) International publication number: WO 2021/129027

(56) References cited:
- EP-A1- 2 460 486
- WO-A1-2018/092071
- WO-A2-2017/152009
- CN-A- 102 198 015
- CN-A- 103 584 911
- CN-A- 103 654 948
- CN-A- 103 908 334
- CN-A- 104 799 937
- US-A1- 2016 324 572
- US-A1- 2016 324 572
- US-A1- 2017 252 101
- US-A1- 2017 252 101

## Description

### TECHNICAL FIELD

The present invention pertains to the field of medical instruments technology, and relates particularly to an ablation system and a nerve detection device thereof.

### BACKGROUND

Ablation, such as radiofrequency (RF) ablation, has been widely used in interventional surgical treatment of various diseases including arrhythmia, refractory hypertension and tumors. An arrhythmia treatment procedure may involve introducing an ablation catheter into the heart through a blood vessel and identifying an aberrant electrical signal pathway or trigger by virtue of endocardial mapping. Ablation may be then performed in the heart to block aberrant electrical signals transmission, thus restoring the patient's normal sinus cardiac rhythm and achieving curative results.

In the field of refractory hypertension treatment, it has been reported to conduct renal artery RF ablation procedures using unipolar or multipolar renal artery RF ablation catheters. Renal artery RF ablation is an interventional technique for local coagulation necrosis and denervation of sympathetic nerves surrounding a renal artery by RF energy applied to a given site of the renal artery by an electrode catheter delivered there through a blood vessel. As RF energy affects only a minor part and does not cause harm to the body, renal artery RF ablation has been recognized as an effective renal sympathetic nerve denervation method. Apart from this, some have suggested that hypertension treatment effects can also be achieved by destroying certain sympathetic nerves in the aortic vessel wall.

An existing RF ablation system that has found extensive use in renal artery RF ablation includes essentially an RF ablator for producing RF energy and an RF ablation catheter for apply the RF energy to a renal artery. The RF ablation catheter is equipped with an electrode, and is configured to be introduced into a patient's body through the femoral artery and then advanced into the renal artery. The RF ablator is then activated to produce RF energy, which is applied by the electrode to a target site to be ablated. For ease of operation, the RF ablator is often equipped with a display panel and a footswitch, and a physician can permit or interrupt the application of RF energy by depressing or releasing the footswitch.

However, this RF ablator in the existing RF ablation system is only able to provide RF energy as required to ablate a target site, and the identification of the ablation site is entirely dependent on the physician's experience and at his/her discretion. Inaccurate identification of an ablation target due to insufficient experience of the physician may lead to excessive ablation or other undesirable surgical effects, which may expose the patient to a high risk.

US 2016/324572 A1 discloses methods for using blood pressure parameters derived from arterial blood pressure waveforms.

### SUMMARY OF THE INVENTION

It is an object of the present invention to provide an ablation system, a nerve detection device and computer-readable storage mediums, which allow detecting-based accurate location of an ablation target and thus enhanced effectiveness and safety of an ablation procedure.

The above object is attained by a nerve detection device according to the present invention, which comprises a computation and control module, an energy generation module and a blood pressure monitoring module. The computation and control module is electrically connected to each of the energy generation module and the blood pressure monitoring module.

The invention is defined in independent claims 1 and 11, further embodiments are described in the dependent claims.

Methods of treatment by surgery described here below are not part of the invention.

The energy generation module is configured to connect to a detecting catheter and to detect a given site in an artery by outputting a first energy to the detecting catheter.

The blood pressure monitoring module is configured to connect to a blood pressure sensor, which is configured to monitor a patient's blood pressure prior to and during the detecting for the given site by the energy generation module with the first energy and to output a blood pressure monitoring result. The blood pressure monitoring module is further configured to transmit the blood pressure monitoring results to the computation and control module.

The computation and control module is configured to determine, from the blood pressure monitoring results, whether there is an ablation target at the given site.

In the nerve detection device, the computation and control module is configured to identify, from the blood pressure monitoring results, a patient's blood pressure variation pattern during the detecting for the given site by the energy generation module with the first energy and to determine that there is an ablation target at the given site if the patient's blood pressure variation pattern is compatible with a pre-defined first blood pressure variation pattern, or determine that there is no ablation target at the given site if the patient's blood pressure variation pattern is compatible with a pre-defined second blood pressure variation pattern.

In the nerve detection device, the pre-defined first blood pressure variation pattern features a continuous rise over a predetermined amount, or a drop followed by a rise beyond a blood pressure baseline, of the patient's blood pressure during a predetermined period of time after the beginning of the detecting for the given site by the energy generation module with the first energy, wherein:
the pre-defined second blood pressure variation pattern features a drop followed by a rise below the blood pressure baseline, a plateau or a continuous drop, of the patient's blood pressure during a predetermined period of time after the beginning of the detecting for the given site by the energy generation module with the first energy; and
the blood pressure baseline is the patient's blood pressure prior to the detecting for the given site by the energy generation module with the first energy.

Optionally, the nerve detection device may further comprise a multi-parameter detection module electrically connected to the computation and control module, the multi-parameter detection module configured to detect one or more of: a temperature, a power and a time during an ablation process,
wherein the computation and control module is further configured to automatically adjust an output energy of the energy generation module based on the detection result of the multi-parameter detection module.

The nerve detection device further comprises an ablation effect prediction module predefined with a plurality of ablation effect assessment items and associated preset weights, wherein
the ablation effect prediction module is configured to: acquire assessment criteria associated with the respective ablation effect assessment items; compare the assessment criteria with the respective ablation effect assessment items; derive a final weight from a comparison result; and obtaining a prediction result by calculating a predicted ablation success rate value based on the final weight.

In the nerve detection device, the ablation effect assessment items include one or more of: whether a blood pressure baseline is high, whether the blood pressure has risen significantly during an ablation, a number of sites where the ablation caused a rise of the blood pressure, and whether any vasodilator has been taken prior to the ablation, wherein the blood pressure baseline is the patient's blood pressure prior to the detecting for the given site by the energy generation module with the first energy.

In the nerve detection device, the predicted ablation success rate value is calculated according to M=N+qN, where M represents the predicted ablation success rate value; N, a preset basic ablation success rate; and q, the final weight.

Optionally, in the nerve detection device, when the computation and control module determines that there is an ablation target at the given site, it may issue an indication that it is suitable to control the energy generation module to ablate the given site with a second energy applied by the detecting catheter, which is higher than the first energy, wherein when the computation and control module determines that there is no ablation target at the given site, it issues an indication that it is suitable to continue or stop detecting.

Optionally, the nerve detection device may further comprise a display module for displaying an image of the given site, wherein the display module is communicatively connected to the computation and control module and is configured to display the indication when the computation and control module determines whether there is ablation target at the given site based on the blood pressure monitoring result of the blood pressure monitoring module, or wherein the display module is communicatively connected to the ablation effect prediction module and is configured to display a prediction result.

Optionally, in the nerve detection device, the indication may be rendered with a text and a color block by displaying a color block associated with the text "Strongly Recommended" or "Recommended" in a predetermined first or second color if the patient's blood pressure variation pattern is compatible with the pre-defined first blood pressure variation pattern or displaying a color block associated with the text "Not Recommended" in a predetermined third color if the patient's blood pressure variation pattern is compatible with the pre-defined second blood pressure variation pattern.

Optionally, in the nerve detection device, the energy generation module may comprise a radiofrequency (RF) energy module configured to provide the first energy which is a low-power ablation energy and the second energy which is a high-power ablation energy.

Optionally, in the nerve detection device, the energy generation module may comprise a stimulation module configured to provide the first energy which is stimulation energy and an RF energy module configured to provide the second energy which is high-power ablation energy.

Optionally, in the nerve detection device, the energy generation module may comprise one or more of: an RF energy module, a pulse energy module, a laser module, an ultrasonic module, a radiation module, an optical energy module and a cryogenic energy module and configured to provide one or more of: RF energy, pulse energy, laser energy, ultrasonic energy, radiation energy, optical energy and cryogenic energy.

Optionally, the nerve detection device may further comprise a power supply module configured to power each of the computation and control module, the energy generation module and the blood pressure monitoring module.

Optionally, in the nerve detection device, the artery may be a renal artery or an aorta.

Based on the same inventive concept, the present invention also provides an ablation system as defined in any of the preceding paragraphs and a detecting catheter, wherein the detecting catheter is an ablation catheter equipped with at least two electrodes.

Based on the same inventive concept, the present invention also provides a computer-readable storage medium having a computer program stored thereon, once executed by a processor, implementing a nerve detecting method comprising:
determining a blood pressure variation pattern based on the monitoring result of a patient's blood pressure; and determining that there is an ablation target at a given site in the patient if the blood pressure variation pattern is compatible with a pre-defined first blood pressure variation pattern, or determining that there is no ablation target at the given site in the patient if the blood pressure variation pattern is compatible with a pre-defined second blood pressure variation pattern,
wherein the pre-defined first blood pressure variation pattern features a continuous rise over a predetermined amount, or a drop followed by a rise beyond a blood pressure baseline, of the patient's blood pressure,
wherein the pre-defined second blood pressure variation pattern features a drop followed by a rise below the blood pressure baseline, a plateau or a continuous drop, of the patient's blood pressure, and
wherein the given site is a part of an artery.

Based on the same inventive concept, the present invention also provides a computer-readable storage medium having a computer program store thereon, once executed by a processor, implementing an ablation effect prediction method comprising: acquiring assessment criteria associated with plurality of respective ablation effect assessment items; comparing the assessment criteria with the respective ablation effect assessment items; deriving a final weight from the comparison result; and obtaining a prediction result by calculating a predicted ablation success rate value based on the final weight.

The ablation effect assessment items include one or more of whether a blood pressure baseline is high, whether the blood pressure has risen significantly during ablation, a number of sites where the ablation caused a rise of the blood pressure, and whether any vasodilator has been taken prior to ablation, wherein the predicted ablation success rate value is calculated according to M=N+qN, where M represents the predicted ablation success rate value; N, a preset basic ablation success rate; and q, the final weight.

Compared to the prior art, the ablation system, the nerve detection device thereof and the computer-readable storage mediums offer the following benefits:
whether an ablation target is present at a given site of interest in an artery of a patient is determined by applying a first energy to the given site and monitoring how the patient's blood pressure varies. This enables accurate location of an ablation target in a sympathetic nerve ablation procedure. Moreover, after an ablation target is located, an indication is automatically given as to ablate the target by outputting an ablation energy. The accurate ablation target location entailed by the present invention overcomes the problems of excessive or ineffective ablation seen in the current practice and avoids the heavy reliance of an ablation procedure on the physician's experience. As a result, an increased cure rate, reduced excessive arterial damage and markedly improved surgical effectiveness and safety are achieved.

### BRIEF DESCRIPTION OF THE DRAWINGS

Fig. 1 is a schematic diagram illustrating the structure of a nerve detection device according to an embodiment of the present invention.
Fig. 2 is a schematic diagram illustrating the structure of a nerve detection device according to another embodiment of the present invention.
Fig. 3 is a flowchart of a nerve detecting method according to an embodiment of the present invention.
Fig. 4 schematically illustrates an area A between two adjacent samples in a blood pressure curve according to an embodiment of the present invention.
Fig. 5 is a schematic illustration of an intelligent ablation indication interface according to an embodiment of the present invention.

### DETAILED DESCRIPTION

The present invention proposes a nerve detection device suitable for use in detecting the presence of an ablation target at a given site in the aorta or a renal artery. When such presence is determined, the nerve detection device is also suitable for use with an ablation catheter in an interventional manner to apply energy to the aorta or renal artery to enable therapeutic treatment of refractory hypertension. Success of such treatment depends on whether the applied energy can block sympathetic nerves surrounding the aorta or renal artery. However, as the distribution of sympathetic nerves varies between individuals, and since there is to date no reliable nerve location method, the current surgical practice in this area substantially has to rely on blind attempts.

The core idea of the present invention is to determine whether there are sympathetic nerves (i.e., an ablation target) at a given site by monitoring blood pressure responses to the application of energy to the site. If the determination is positive, a destructive ablation action is taken at the site to destroy the sympathetic nerves there. Otherwise, no additional ablation is necessary for the site. This results in improved surgical safety and effectiveness. More specifically, in an aorta or renal artery ablation procedure, a first energy is used to detect a given site, and a blood pressure response thereto is concurrently monitored in real time. If a blood pressure change that meets a predetermined criterion is monitored, then it is determined that there is an ablation target present at the given site, and a second energy is then applied to the given site to ablate it. This allows accurate location of an ablation target in an ablation procedure and avoids excessive ablation or other undesirable surgical effects, resulting in better surgical results.

Objects, advantages and features of the present invention will become more apparent from the following detailed description of illustrated embodiments thereof, which is to be read in connection with the accompanying drawings.

In addition to energy generation and power supply modules common to the existing nerve detection devices, the proposed nerve detection device further includes a blood pressure monitoring module having an external or built-in blood pressure sensor capable of real-time blood pressure monitoring during operation and a computation and control module configured to determine the position of an ablation target from blood pressure monitoring results of the blood pressure monitoring module and control an output energy level of the energy generation module.

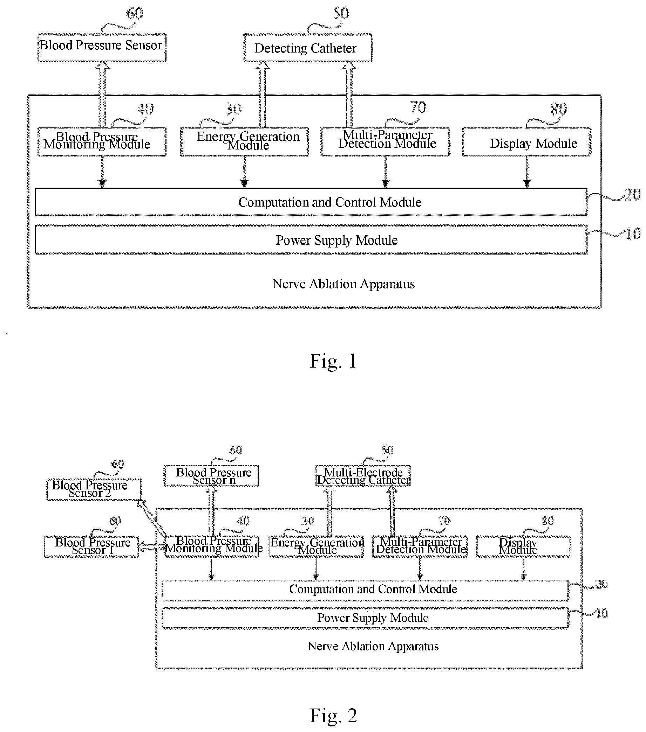

Reference is now made to Fig. 1, a schematic diagram illustrating the structure of a nerve detection device according to an embodiment of the present invention. The nerve detection device according to this embodiment includes a power supply module 10, a computation and control module 20, an energy generation module 30 and a blood pressure monitoring module 40. The power supply module 10 is configured to power each of the computation and control module 20, the energy generation module 30 and the blood pressure monitoring module 40. The computation and control module 20 is electrically connected to each of the energy generation module 30 and the blood pressure monitoring module 40.

In alternative embodiments, the power supply module may be replaced with a power supply interface configured to connect to an external power supply, and the blood pressure monitoring module may be replaced with a blood pressure signal interface for connecting an external blood pressure sensor. However, the present invention is not so limited.

The energy generation module 30 is configured to connect to a detecting catheter 50 and to detect a given site in an artery by outputting a first energy to the given site. Non-limiting examples of the artery may include the aorta and the renal arteries. Specifically, the energy generation module 30 may apply the energy to the given site through the detecting catheter 50. The first energy is at a relatively low level suitable for target detecting that enables accurate location of an ablation target. In this embodiment, the first energy is used as a stimulus and usually measured in milliampere-volts. A particular form of the energy may be an impulse capable of applying an electrical stimulus to the given site, which does not cause damage to cells at the site but induces a change in a blood pressure of the patient. The energy generation module 30 may further include a stimulation module for providing the first energy as a stimulus to the given site in the artery, which enables location of an ablation target.

In alternative embodiments, the stimulation module may be replaced with a stimulation signal interface configured to connect to an external stimulator. However, the present invention is not so limited.

In this embodiment, the blood pressure monitoring module 40 is configured to connect to a blood pressure sensor 60 configured to monitor the blood pressure of the patient prior to and during the detecting for the given site with the first energy by the energy generation module 30 and to transmit a monitoring result to the blood pressure monitoring module 40. In particular, the blood pressure monitoring module 40 may be able to monitor the blood pressure in real time during a procedure and output the blood pressure monitoring results in the form of a blood pressure curve or blood pressure values, which serves as a basis for the computation and control module 20 to make a determination.

The computation and control module 20 is configured to determine, based on the blood pressure monitoring results from the blood pressure monitoring module 40, whether there is an ablation target present at the given site. If so, then it provides the physician with an indication that it is suitable to control the energy generation module 30 to ablate the given site with a second energy. If not, then it provides the physician with an indication that he/she may continue or stop detecting. The second energy is higher than the first energy. The second energy may be a high-power ablation energy measured in watts. Here, the "high-power ablation energy" is meant to refer to a level of energy that, when applied to the given site, can cause destructive damage and death of cells there. The high-power ablation energy is generally in the range of 5-20 W and may vary depending on conditions of the patient. Preferably, the nerve detection device further has an information input module allowing the physician to modify or set a value of the high-power ablation energy.

The energy generation module 30 may further include a radiofrequency (RF) energy module or be connected to an external RF ablator. The RF energy module may be configured to provide the second energy for ablating the given site in the artery. In this embodiment, as an ablation catheter, the detecting catheter 50 is preferably provided with two electrodes for delivering the first energy. Additionally, the second energy may be delivered by one of the electrodes.

In alternative embodiments, the RF energy module may be replaced with an ablation signal interface configured to connect to an RF ablator. However, the present invention is not so limited.

In a preferred embodiment, the first energy is a low-power ablation energy measured in watts. Unlike stimulation energy, ablation energy acts on the given site in the form of a sine wave. Here, the low-power ablation energy is meant to refer to a level of energy that, when applied to the given site, does not cause damage to cells at the given site but induces a change in the patient's blood pressure. The low-power ablation energy is less than 5 W, preferably 2 W. Likewise, it also varies depending on conditions of the patient, and its value can also be modified or set by the physician through the information input module. Since the second energy is a high-power ablation energy, in this embodiment, when it is determined that there is an ablation target at the given site from the detecting with the low-power ablation energy, without needing to switch to another energy source, the high-power ablation energy can be obtained by directly tuning up the output level of the same energy source. In this way, both the first energy and the second energy are provided by the RF energy module of the energy generation module 30, resulting in improved surgical efficiency, reduced surgical complexity and a shortened surgical time.

In this embodiment, the computation and control module 20 may automatically perform a calculation based on a continuous blood pressure curve or discrete blood pressure values acquired by the blood pressure monitoring module 40 to determine whether there is an ablation target present at the given site and accordingly control activation/deactivation of the energy generation module 30 or a level of output energy therefrom. The computation and control module 20 may further include a blood pressure variation determination module and a power control module. The blood pressure variation determination module may be configured to automatically perform a calculation based on a continuous blood pressure curve or discrete blood pressure values acquired by the blood pressure monitoring module 40 to determine whether there is an ablation target present at the given site. The power control module may be configured to control the energy generation module to output an accordingly appropriate level of energy.

More specifically, the computation and control module 20 may follow the following steps to determine whether there is an ablation target at the given site from the blood pressure monitoring results of the blood pressure monitoring module 40: identifying, from the blood pressure monitoring results of the blood pressure monitoring module 40, a variation pattern of the patient's blood pressure in response to the detecting for the given site by the energy generation module 30 with the first energy; and determining that there is an ablation target at the given site if the variation pattern of the patient's blood pressure is compatible with a pre-defined first blood pressure variation pattern, or determining that there is no ablation target at the given site if the variation pattern of the patient's blood pressure is compatible with a pre-defined second blood pressure variation pattern.

The pre-defined first blood pressure variation pattern may feature a continuous rise over a predetermined amount, or a drop followed by a rise beyond a blood pressure baseline, of the patient's blood pressure during a predetermined period of time after the beginning of the detecting for the given site by the energy generation module 30 with the first energy.

The pre-defined second blood pressure variation pattern may feature a drop followed by a rise below the blood pressure baseline, a plateau or a continuous drop, of the patient's blood pressure during a predetermined period of time after the beginning of the detecting for the given site by the energy generation module 30 with the first energy.

The blood pressure baseline may be a value of the patient's blood pressure prior to the detecting for the given site by the energy generation module 30 with the first energy.

The nerve detection device may further include a multi-parameter detection module 70 configured for one or more of temperature monitoring, power monitoring and time monitoring during an ablation process. In this case, the computation and control module 20 may be further configured to automatically adjust output energy from the energy generation module 30 based on a detection result of the multi-parameter detection module 70.

Preferably, the nerve detection device may further comprise a display module 80 for displaying an image for the given site to the physician when the computation and control module 20 is determining whether there is an ablation target at the given site and/or indication information from the computation and control module 20, such as a recommendation of using the second energy to ablate the given site. Displaying the location of the ablation target on the display module 80 during an ablation procedure can provide the physician with more information that is helpful in improving the effectiveness and safety of the ablation procedure. The display module 80 may be replaced with a display signal interface configured to connect to an external display device, but the present invention is not so limited.

Preferably, the nerve detection device is a multi-channel nerve detection device as shown in Fig. 2, in which the blood pressure monitoring module 40 is coupled to multiple blood pressure sensors 60 and the energy generation module 30 is coupled to a multi-electrode detecting catheter 50. In this case, multi-point monitoring of the patient's blood pressure is made possible prior to and during the detecting of multiple given sites by the energy generation module 30 with the first energy. In other words, the blood pressure monitoring module 40 in the nerve detection device may be a multi-channel module, and the detecting catheter 50 may be accordingly equipped preferably with three or more electrodes capable of detecting an equal number of given sites at the same time. This allows multi-point monitoring and simultaneous ablation, which results in savings in time required for ablation target location and ablation.

Monitoring the blood pressure at multiple locations enables increased reliability in blood pressure monitoring. For example, certain blood vessels of the patient may be clotted or diseased (e.g., vasculitis), and only monitoring a single location may lead to inaccurate blood pressure monitoring results, which may adversely affect the identification of the variation pattern. Examples of monitored locations may include the femoral, brachial, superficial temporal, axillary and other arteries. For an identified target, simultaneous multi-point ablation with multiple electrodes on the detecting catheter 50 enables easier creation of a circular, square, linear or similar ablation lesion with a reliable blocking effect.

In the multi-channel nerve detection device, the computation and control module 20 may determine, from multi-point blood pressure monitoring results of the blood pressure monitoring module 40, whether there is any ablation target at the monitored given sites. More specifically, the computation and control module 20 may process the multi-point blood pressure monitoring results transmitted from the blood pressure monitoring module 40 to derive an overall blood pressure variation pattern and then determine whether this blood pressure variation pattern is compatible with any of the pre-defined blood pressure variation patterns. In this way, the multi-channel nerve detection device is capable of quick, accurate location of multiple ablation targets by deriving a variation rate of the blood pressure, which indicates the presence of the targets, from blood pressure values measured at the multiple locations. Moreover, it may control the energy generation module to output energy simultaneously to the multiple targets, thus achieving quick treatment within a shortened surgical time.

Preferably, the nerve detection device may further include an ablation effect prediction module predefined with a plurality of ablation effect assessment items and associated preset weights. The ablation effect prediction module is configured to acquire assessment criteria associated with the respective ablation effect assessment items, compare the assessment criteria with the respective ablation effect assessment items, derive a final weight from both the comparison results and the preset weights, and obtain a prediction result by calculating a predicted ablation success rate value based on the final weight.

The ablation effect assessment items may include whether the blood pressure baseline is high, whether the blood pressure has risen significantly during ablation, a number of sites where the ablation caused a rise of the blood pressure, and whether any vasodilator has been taken prior to ablation. Here, the blood pressure baseline may be a value of the patient's blood pressure prior to the detecting for the given site by the energy generation module with the first energy.

Therefore, the ablation effect prediction module is capable of predicting the surgical results. More specifically, the physician may enter multiple assessment criteria to the ablation effect prediction module using the information input unit of the device. The ablation effect prediction module may then compare the assessment criteria with the parameter conditions of corresponding ablation effect assessment items and derive the final weight and thus the prediction result from the comparison results. The ablation effect prediction module may automatically acquire some parameters in the varying blood pressure as assessment criteria from the blood pressure variation determination module and compare these and the parameter conditions of the corresponding ablation effect assessment items. Then, together with the former and posterior comparison results, it may derive the prediction result.

The prediction result may be derived from a basic ablation success rate and a weight which can be pre-stored in the ablation effect prediction module or determined by the physician based on his/her own experience and input to the ablation effect prediction module using the information input unit. An example of this is shown in Table 1 below.

**Table 1**

| Ablation Effect Assessment Item | Parameter Condition | Weight |
|---|---|---|
| Is the blood pressure baseline high? | Blood pressure baseline>160mmHg | 0.3 |
| Has the blood pressure risen significantly during ablation? | P>40 mmg, where P=[(P1-P0)+...(pn-p0)]/n, and n is the number of ablations performed | 0.3 |
| What is the number of sites where the ablation caused a rise of the blood pressure? | m>k*80%, where m=Count(Px>Px-1, x=1,2, ..., n), k is the total number of ablations performed, and m is the number of ablations caused a rise of the blood pressure | 0.2 |
| Has any vasodilator been taken prior to ablation? | Taken | 0.2 |

The above information may be manually entered via a human-computer interaction interface of the nerve detection device or automatically acquired by software, and the ablation effect prediction result can be derived based on predefined criteria and automatically displayed on the display module 80. The predicted ablation success rate value M may be calculated according to M=N+qN, where N represents the basic ablation success rate provided by the physician and q is the final weight determined from the ablation effect assessment items and preset weights listed in Table 1 (which is the sum of the weights whose parameter conditions are satisfied). For example, assuming that the basic ablation success rate is 50% and that the assessment criteria satisfy the parameter conditions of the first and second items in Table 1, the final weight is 0.6=(0.3+0.3), and the ablation success rate is improved to 50%+0.6*50%=80%. Other examples are also possible.

In this embodiment, the blood pressure sensor 60 may be either invasive or non-invasive, with the former being preferred. An invasive blood pressure meter with a universal interface, such as a PVB interface, can be suitably used. The detecting catheter 50 is an interventional catheter serving as an energy transfer medium. Any detecting catheter 50 may be used in the nerve detection device, optionally with the aid of an adapter.

The energy generation module 30 may further include one or more of a pulse energy module, a laser module, an ultrasonic module, a radiation module, an optical energy module and a cryogenic energy module so as to able to provide one or more of pulse energy, laser energy, ultrasonic energy, radiation energy, optical energy and cryogenic energy.

A nerve detecting method proposed in the present invention will be described in detail below.

In embodiments of the present invention, there is provided a nerve detecting method for use with the nerve detection device as defined above.

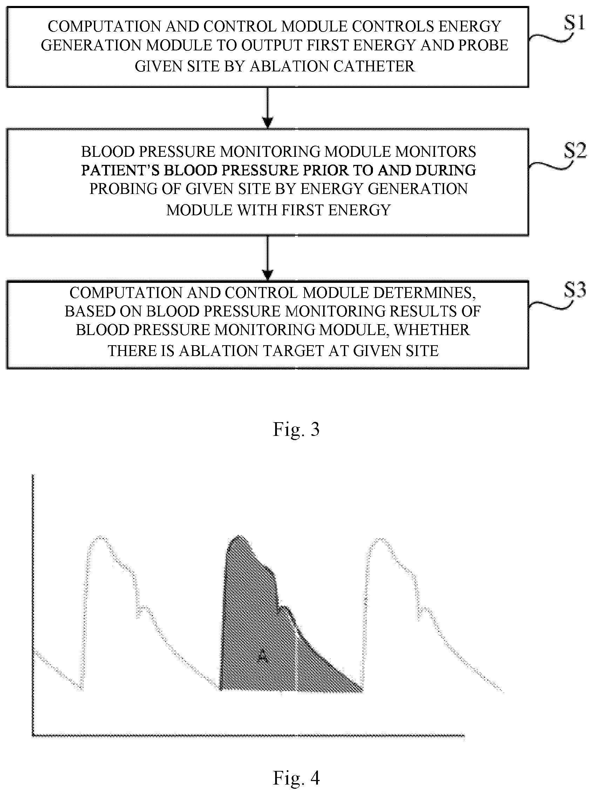

Reference is now made to Fig. 3, a flowchart of the nerve detecting method according to an embodiment of the present invention. In this embodiment, the nerve detecting method includes the steps detailed below.

In step S1, the computation and control module controls the energy generation module to output a first energy so that the ablation catheter detects a given site.

In this embodiment, the energy generation module outputs low-power ablation energy (e.g., not exceeding 5 W).

In step S2, the blood pressure monitoring module monitors the patient's blood pressure prior to and during the detecting for the given site by the energy generation module with the first energy.

A blood pressure value obtained from the monitoring of the patient's blood pressure prior to the detecting for the given site by the energy generation module with the first energy may be taken as a blood pressure baseline, and provided, together with monitored values indicating variation of the patient's blood pressure during the detecting for the given site by the energy generation module with the first energy, to the computation and control module for calculation and decision-making.

In this embodiment, the monitored blood pressure may be the maximum systolic pressure. The blood pressure baseline P0 may be the patient's blood pressure measured without the first energy being output. After the detecting for the given site by the energy generation module with the first energy begins, the patient's blood pressure may be monitored for a predetermined period of time T, which may last until the blood pressure does not exhibit variation anymore and is typically 5 minutes, preferably 3 minutes. The blood pressure monitoring may be conducted at a sampling frequency of n, which may be set as n=T*60/10 beforehand when the monitoring is invasive. In this case, an average blood pressure value within the time period T. If the monitoring is conducted using a blood pressure sensor, then T should be designed to be 3-5 times a minimum possible sampling interval of the blood pressure sensor and not exceed 10 minutes.

In step S3, the computation and control module determines, from the blood pressure monitoring results of the blood pressure monitoring module, whether there is an ablation target at the given site. If the presence is determined, it issues an indication it is suitable to control the energy generation module to ablate the given site with second energy from the detecting catheter. Otherwise, it issues an indication to continue or stop detecting. The second energy may be output from the energy generation module at a power level of 5-100 W, which enables nerve blockade while avoiding any trauma.

More specifically, in step S3, the determination of whether there is an ablation target at the given site by the computation and control module based on the blood pressure monitoring results from the blood pressure monitoring module may include:
identifying, by the computation and control module, a variation pattern of the patient's blood pressure during the detecting for the given site by the energy generation module with the first energy based on the blood pressure monitoring results of the blood pressure monitoring module; and
determining that there is an ablation target at the given site if the variation pattern of the patient's blood pressure is compatible with a pre-defined first blood pressure variation pattern, or determining that there is no ablation target at the given site if the patient's blood pressure variation pattern is compatible with a pre-defined second blood pressure variation pattern.

The pre-defined first blood pressure variation pattern may feature a persistent elevation over a predetermined amount, or a drop followed by a rise beyond the blood pressure baseline, of the patient's blood pressure during the predetermined period of time after the beginning of the detecting for the given site by the energy generation module with the first energy.

The pre-defined second blood pressure variation pattern may feature a drop followed by a rise below the blood pressure baseline, a plateau or a continuous drop, of the patient's blood pressure during a predetermined period of time after the beginning of the detecting for the given site by the energy generation module with the first energy.

The blood pressure baseline (i.e., P0) may be a value of the patient's blood pressure prior to the stimulation or ablation for the given site by the energy generation module with the first energy. The predetermined amount *a* may be within the range of 5-30 mmHg and is preferably 10 mmHg or more. This can also be modified or set according to low-power ablation energy information entered by the physician through the information input module.

The five characteristic behaviors for determining the presence or absence of an ablation target, i.e., the above-described two featured by the first blood pressure variation pattern and three by the second blood pressure variation pattern, may be pre-configured in the computation and control module. Upon receiving the blood pressure monitoring results from the blood pressure monitoring module, the computation and control module may identify a blood pressure variation pattern based on both the blood pressure baseline and blood pressure variation within the predetermined period of time T. If the blood pressure variation exhibits either characteristic behavior of the first blood pressure variation pattern, it is determined that there is an ablation target at the given site, and it is necessary to tune up the output power of the energy generation module to ablate the given site. Otherwise, if the blood pressure variation exhibits any characteristic behavior of the second blood pressure variation pattern, then it is determined that there is no ablation target at the given site, and it is recommended to not ablate the given site and cause the energy generation module stop outputting power.

Table 2 summarizes the five characteristic behaviors, among which the first two are of the first blood pressure variation pattern, and the remaining three are of the second blood pressure variation pattern. As shown in Table 2, the two characteristic behaviors of the first blood pressure variation pattern respectively include a continuous rise over the predetermined amount *a* and a drop followed by a rise beyond the blood pressure baseline. It would be appreciated that, form a continuous rise of blood pressure, an initial determination can be made as to the presence of sympathetic nerves that qualify the given site as an ablation target. However, in practice, as some sympathetic nerves respond slowly, or due to the presence of parasympathetic nerves, following the application of the first energy, the blood pressure may drop first due to the presence of a sympathetic nerve before it rises due to the presence of sympathetic nerves. On the other hand, even when a continuous blood pressure rise occurs, if the total amount of rise is insignificant, it may indicate that there are only a few sympathetic nerves at the given site that are insufficient to qualify the site as an ablation target.

Therefore, the reliance simply on a continuous rise of blood pressure may lead to missed diagnosis in scenarios with co-presence of sympathetic and parasympathetic nerves, making the ablation target identification inaccurate. In view of this, this embodiment further specifies an amount of rise that exceeds the predetermined amount *a* in addition to a continuous rise of blood pressure in the first variation pattern, and adds a second variation pattern that specifies a drop of blood pressure followed by a rise beyond the blood pressure baseline and thus takes into account the scenarios with co-presence of sympathetic and parasympathetic nerves. This allows more accurate identification of an ablation target by comprehensively taking into account all possible scenarios and overcomes the drawbacks associated with reliance simply on a continuous blood pressure rise.

**Table 2**

| No. | Blood Pressure Variation Pattern within a Period of Time | Intelligent Indication on Interface of Display Module | Output Power Control |
|---|---|---|---|
| 1 | Continuous rise over the predetermined amount *a* | Target ablation strongly recommended by light, sound or other means | Increase the output power to a level as required by ablation |
| 2 | Drop followed by a rise beyond the blood pressure baseline | Target ablation recommended by light, sound or other means | Increase the output power to a level as required by ablation |
| 3 | Drop followed by a rise below the blood pressure baseline | Ablation not recommended | Stop power output |
| 4 | Plateau | Ablation not recommended | Stop power output |
| 5 | Continuous Drop | Ablation not recommended | Stop power output |

The blood pressure variation pattern may be identified based on multiple blood pressure values continuously sampled across the predetermined period of time T, or base on a fitted curve thereof. In the former case, for example, if the multiple blood pressure values continuously sampled across the predetermined period of time T satisfy Px>Px-1(x=1,2,...,n) and (Pn-P0)>a, then a blood pressure variation pattern with a continuous rise over the predetermined amount *a* can be identified.

In the latter case, areas A between adjacent samples in the blood pressure curve may be calculated and sorted in the order of the samples, and a blood pressure variation pattern can be identified based on the variation of area A. For example, if the areas *A* satisfy Ax>Ax-1(x=1,2,...,n) and (An-A0)>a, where A0 represents an area between adjacent samples in the blood pressure baseline, then a blood pressure variation pattern with a continuous rise over the predetermined amount *a* can be identified. For ease of understanding, Fig. 4 shows an area A between two adjacent samples in the blood pressure curve. It would be appreciated that each area A can be calculated by integration.

Preferably, when the nerve detection device further includes the ablation effect prediction module, the nerve detecting method may further include the steps below.

In step S4, the ablation effect prediction module provides an ablation effect prediction during ablation for the given site by the energy generation module with the second energy.

In step S5, the computation and control module control the energy generation module to continue or stop ablation according to the ablation effect prediction.

Further, in case of the nerve detection device further including the display module, the nerve detecting method may further include: in step S4, automatically displaying the ablation effect prediction of the ablation effect prediction module on the display module.

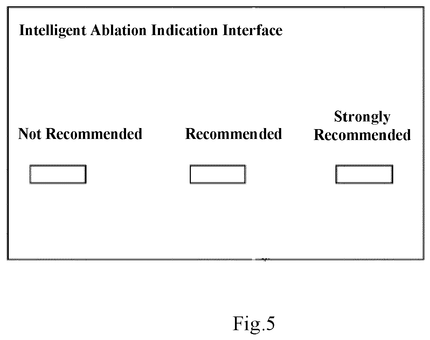

Additionally, in step S3, an image of the given site may also be displayed on the display module, and in the course of the computation and control module determining whether there is an ablation target at the given site based on the blood pressure monitoring results of the blood pressure monitoring module, an indication of whether it is recommended to ablate the given site may be displayed on the display module in real time. As shown in Fig. 5, on an indication interface of the display module in the nerve detection device, text may be used in combination with color blocks to indicate in real time whether ablation of the given site is recommended based on a determination made on the basis of the variation of blood pressure. The color blocks may be displayed under the text. For example, if ablation of the given site is strongly recommended, then the color block under the text "Strongly Recommended" may be displayed in a first color, for example, red. If ablation of the given site is recommended, the color block under the text "Recommend" may be displayed in a second color, for example, yellow. If ablation of the given site is not recommended, the color block under the text "Not Recommend" may be displayed in a third color, for example, green. For example, as shown in Table 2, the given site may be strongly recommended for ablation when the patient's blood pressure variation pattern is compatible with the No. 1 first blood pressure variation pattern, or recommended for ablation when the patient's blood pressure variation pattern is compatible with the No. 2 first blood pressure variation pattern, or not recommended for ablation when the patient's blood pressure variation pattern is compatible with the No. 3, 4 or 5 second blood pressure variation pattern.

The indication of whether it is recommended to continue ablating the given site and the ablation effect prediction displayed on the display module can help the physician complete the ablation procedure by quickly providing him/her important information that indicates the accurate location of the ablation target and informs the physician of whether the ablation is likely to succeed or not in a real-time way.

The present invention also provides a computer-readable storage medium storing a computer program, which when executed by a processor, implements a nerve detecting method comprising:
determining a blood pressure variation pattern based on the monitoring results of a patient's blood pressure; and determining that there is an ablation target at a given site in the patient if the blood pressure variation pattern is compatible with a pre-defined first blood pressure variation pattern, or determining that there is no ablation target at the given site in the patient if the blood pressure variation pattern is compatible with a pre-defined second blood pressure variation pattern,
wherein the pre-defined first blood pressure variation pattern features a continuous rise over a predetermined amount, or a drop followed by a rise beyond a blood pressure baseline, of the patient's blood pressure,
wherein the pre-defined second blood pressure variation pattern features a drop followed by a rise below the blood pressure baseline, a plateau or a continuous drop, of the patient's blood pressure, and
wherein the given site is part of an artery.

The present invention also provides another computer-readable storage medium storing a computer program, which when executed by a processor, implements an ablation effect prediction method comprising: acquiring assessment criteria associated with plurality of respective ablation effect assessment items; comparing the assessment criteria with the respective ablation effect assessment items; deriving a final weight from based on comparison results and preset weight; and obtaining a prediction result by calculating a predicted ablation success rate value based on the final weight.

Optionally, the ablation effect assessment items may include one or more of whether a blood pressure baseline is high, whether there is a significant blood pressure rise during ablation, a number of sites where the ablation caused a rise of the blood pressure, and whether any vasodilator has been taken prior to ablation, wherein the predicted ablation success rate value is calculated according to M=N+qN, where M represents the predicted ablation success rate value; N represents preset basic ablation success rate; and q represents the final weight.

In summary, according to the present invention, whether an ablation target is present at a given site of interest in an artery of a patient is determined by applying first energy to the given site and monitoring how the patient's blood pressure varies. This enables accurate location of an ablation target in a sympathetic nerve ablation procedure. Moreover, after an ablation target is located, an indication is automatically given as to whether it is suitable to ablate the target or not, and an output ablation energy can be accordingly controlled to complete the ablation procedure. The accurate ablation target location entailed by the present invention overcomes the problems of excessive or ineffective ablation seen in the current practice and avoids the heavy reliance of an ablation procedure on the physician's experience. As a result, an increased cure rate, reduced excessive arterial damage and markedly improved surgical effectiveness and safety are achieved.

As used herein, relational terms such as first and second, etc., are only used to distinguish one entity or operation from another entity or operation, and do not necessarily require or imply these entities having such an order or sequence. Moreover, the terms "include," "including," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.

The description presented above is merely that of some preferred embodiments of the present invention and does not limit the scope thereof in any sense. The scope of the invention is defined by the appended claims.

## Claims

1. A nerve detection device, comprising a computation and control module (20), an energy generation module (30), a blood pressure monitoring module (40) and an ablation effect prediction module predefined with a plurality of ablation effect assessment items and associated preset weights, the computation and control module (20) electrically connected to each of the energy generation module (30) and the blood pressure monitoring module (40),
(S1) wherein the energy generation module (30) is configured to connect to a detecting catheter (50) and to detect a given site in an artery by outputting a first energy to the detecting catheter (50),
(S2) wherein the blood pressure monitoring module (40) is configured to connect to a blood pressure sensor (60), wherein the blood pressure sensor (60) is configured to monitor a patient's blood pressure prior to and during the detecting for the given site by the energy generation module (30) with the first energy, and to output a blood pressure monitoring result, and wherein the blood pressure monitoring module (40) is further configured to transmit the blood pressure monitoring result to the computation and control module (20),
(S3) wherein the computation and control module (20) is configured to determine whether an ablation target is present at the given site based on the blood pressure monitoring result and to identify, based on the blood pressure monitoring result, a patient's blood pressure variation pattern during the detecting for the given site by the energy generation module (30) with the first energy, and to determine that an ablation target is present at the given site if the patient's blood pressure variation pattern is compatible with a pre-defined first blood pressure variation pattern, or to determine that no ablation target is present at the given site if the patient's blood pressure variation pattern is compatible with a pre-defined second blood pressure variation pattern,
wherein the pre-defined first blood pressure variation pattern features a continuous rise over a predetermined amount, or a drop followed by a rise beyond a blood pressure baseline, of the patient's blood pressure during a predetermined period of time after beginning of the detecting for the given site by the energy generation module (30) with the first energy,
wherein the pre-defined second blood pressure variation pattern features a drop followed by a rise below the blood pressure baseline, a plateau or a continuous drop, of the patient's blood pressure during a predetermined period of time after beginning of the detecting for the given site by the energy generation module (30) with the first energy,
wherein the blood pressure baseline is the patient's blood pressure prior to the detecting for the given site by the energy generation module (30) with the first energy,
wherein the ablation effect prediction module is configured to: acquire assessment criteria associated with respective ablation effect assessment items; compare the assessment criteria with the respective ablation effect assessment items; derive a final weight based on the comparison result, and obtain a prediction result by calculating a predicted ablation success rate value based on the final weight,
wherein the ablation effect assessment items include one or more of: whether a blood pressure baseline is high, whether the blood pressure has risen significantly during an ablation, a number of sites where the ablation caused a rise of the blood pressure, and whether any vasodilator has been taken prior to the ablation, and
wherein the predicted ablation success rate value is calculated according to M=N+qN, where M represents the predicted ablation success rate value; N represents a preset basic ablation success rate; and q represents the final weight.

2. The nerve detection device of claim 1, further comprising a multi-parameter detection module (70) electrically connected to the computation and control module (20), wherein the multi-parameter detection module (70) is configured to detect one or more of: a temperature, a power and a time during an ablation process,
wherein the computation and control module (20) is further configured to automatically adjust an output energy of the energy generation module (30) based on the detection result of the multi-parameter detection module (70).

3. The nerve detection device of claim 1, wherein when the computation and control module (20) determines that an ablation target is present at the given site, the computation and control module (20) issues an indication of controlling the energy generation module (30) to ablate the given site with a second energy applied by the detecting catheter (50), wherein the second energy is higher than the first energy, and wherein when the computation and control module (20) determines that no ablation target is present at the given site, the computation and control module (20) issues an indication of continue or stop detecting.

4. The nerve detection device of claim 3, further comprising a display module (80) for displaying an image of the given site, wherein the display module (80) is communicatively connected to the computation and control module (20) and is configured to display the indication when the computation and control module (20) determines whether the ablation target is present at the given site based on the blood pressure monitoring result of the blood pressure monitoring module (40), or wherein the display module (80) is communicatively connected to an ablation effect prediction module and is configured to display a prediction result.

5. The nerve detection device of claim 4, wherein the indication is rendered with a text and a color block, presented in such a manner that: displaying a color block associated with the text "Strongly Recommended" or "Recommended" in a predetermined first color or a predetermined second color if a patient's blood pressure variation pattern is compatible with a pre-defined first blood pressure variation pattern; or displaying a color block associated with the text "Not Recommended" in a predetermined third color if a patient's blood pressure variation pattern is compatible with a pre-defined second blood pressure variation pattern.

6. The nerve detection device of claim 3, wherein the energy generation module (30) comprises a radiofrequency (RF) energy module configured to provide the first energy which is a low-power ablation energy and the second energy which is a high-power ablation energy.

7. The nerve detection device of claim 3, wherein the energy generation module (30) comprises a stimulation module configured to provide the first energy which is a stimulation energy, and an RF energy module configured to provide the second energy which is a high-power ablation energy.

8. The nerve detection device of claim 1, wherein the energy generation module (30) comprises one or more of: an RF energy module, a pulse energy module, a laser module, an ultrasonic module, a radiation module, an optical energy module and a cryogenic energy module, and is configured to provide one or more of: an RF energy, a pulse energy, a laser energy, an ultrasonic energy, a radiation energy, an optical energy and a cryogenic energy.

9. The nerve detection device of claim 1, further comprising a power supply module (10) configured to power each of the computation and control module (20), the energy generation module (30) and the blood pressure monitoring module (40).

10. The nerve detection device of claim 1, wherein the artery is a renal artery or an aorta.

11. A computer-readable storage medium having a computer program stored thereon, once executed by a processor of the nerve detection device according to claim 1, implementing an ablation effect prediction method comprising: acquiring assessment criteria associated with a plurality of ablation effect assessment items; comparing the assessment criteria with respective ablation effect assessment items; deriving a final weight based on the comparison result; and obtaining a prediction result by calculating a predicted ablation success rate value based on the final weight, and
identifying, based on the blood pressure monitoring result, a patient's blood pressure variation pattern during the detecting for a given site by an energy generation module with a first energy, and determining that an ablation target is present at the given site if the patient's blood pressure variation pattern is compatible with a pre-defined first blood pressure variation pattern, or determining that no ablation target is present at the given site if the patient's blood pressure variation pattern is compatible with a pre-defined second blood pressure variation pattern,
wherein the pre-defined first blood pressure variation pattern features a continuous rise over a predetermined amount, or a drop followed by a rise beyond a blood pressure baseline, of the patient's blood pressure during a predetermined period of time after beginning of the detecting for the given site by the energy generation module with the first energy,
wherein the pre-defined second blood pressure variation pattern features a drop followed by a rise below the blood pressure baseline, a plateau or a continuous drop, of the patient's blood pressure during a predetermined period of time after beginning of the detecting for the given site by the energy generation module (30) with the first energy,
wherein the blood pressure baseline is the patient's blood pressure prior to the detecting for the given site by the energy generation module (30) with the first energy,
wherein the ablation effect assessment items include one or more of: whether a blood pressure baseline is high, whether the blood pressure has risen significantly during ablation, a number of sites where the ablation caused a rise of the blood pressure, and whether any vasodilator has been taken prior to ablation, and wherein the predicted ablation success rate value is calculated according to M=N+qN, where M represents the predicted ablation success rate value; N represents a preset basic ablation success rate; and q represents the final weight.

## Patentansprüche

1. Eine Nervendetektionsvorrichtung, umfassend ein Berechnungs- und Steuermodul (20), ein Energieerzeugungsmodul (30), ein Blutdrucküberwachungsmodul (40) und ein Ablationseffekt-Vorhersagemodul, das mit einer Vielzahl von Ablationseffekt-Bewertungselementen und zugeordneten voreingestellten Gewichten vordefiniert ist, wobei das Berechnungs- und Steuermodul (20) elektrisch mit jeweils dem Energieerzeugungsmodul (30) und dem Blutdrucküberwachungsmodul (40) verbunden ist,
(S1) wobei das Energieerzeugungsmodul (30) ausgebildet ist, mit einem Detektionskatheter (50) verbunden zu werden und eine bestimmte Stelle in einer Arterie durch Ausgabe einer ersten Energie an den Detektionskatheter (50) zu detektieren,
(S2) wobei das Blutdrucküberwachungsmodul (40) ausgebildet ist, mit einem Blutdrucksensor (60) verbunden zu werden, wobei der Blutdrucksensor (60) ausgebildet ist, den Blutdruck eines Patienten vor und während der Erfassung an der gegebenen Stelle durch das Energieerzeugungsmodul (30) mit der ersten Energie zu überwachen und ein Blutdrucküberwachungsergebnis auszugeben, und wobei das Blutdrucküberwachungsmodul (40) ferner ausgebildet ist, das Blutdrucküberwachungsergebnis an das Berechnungs- und Steuermodul (20) zu übermitteln,
(S3) wobei das Berechnungs- und Steuermodul (20) ausgebildet ist, basierend auf dem Blutdrucküberwachungsergebnis festzustellen, ob an der gegebenen Stelle ein Ablationsziel vorhanden ist, und basierend auf dem Blutdrucküberwachungsergebnis ein Blutdruckvariationsmuster eines Patienten während der Detektion an der gegebenen Stelle durch das Energieerzeugungsmodul (30) mit der ersten Energie zu identifizieren, und festzustellen, dass an der gegebenen Stelle ein Ablationsziel vorhanden ist, wenn das Blutdruckvariationsmuster des Patienten mit einem vordefinierten ersten Blutdruckvariationsmuster kompatibel ist, oder festzustellen, dass an der gegebenen Stelle kein Ablationsziel vorhanden ist, wenn das Blutdruckvariationsmuster des Patienten mit einem vordefinierten zweiten Blutdruckvariationsmuster kompatibel ist,
wobei das vordefinierte erste Blutdruckvariationsmuster einen kontinuierlichen Anstieg über einen vorbestimmten Betrag oder einen Abfall, gefolgt von einem Anstieg über eine Blutdruck-Basislinie hinaus, des Blutdrucks des Patienten während eines vorbestimmten Zeitraums nach Beginn der Detektion der vorgegebenen Stelle durch das Energieerzeugungsmodul (30) mit der ersten Energie aufweist,
wobei das vordefinierte zweite Blutdruckvariationsmuster einen Abfall, gefolgt von einem Anstieg unterhalb der Blutdruck-Basislinie, ein Plateau oder einen kontinuierlichen Abfall des Blutdrucks des Patienten während eines vorbestimmten Zeitraums nach Beginn der Detektion der vorgegebenen Stelle durch das Energieerzeugungsmodul (30) mit der ersten Energie aufweist,
wobei die Blutdruck-Basislinie der Blutdruck des Patienten vor der Detektion der vorgegebenen Stelle durch das Energieerzeugungsmodul (30) mit der ersten Energie ist,
wobei das Ablationseffekt-Vorhersagemodul ausgebildet ist: Bewertungskriterien zu erfassen, die jeweils mit entsprechenden Ablationseffekt-Bewertungselementen assoziiert sind; die Bewertungskriterien mit den jeweiligen Ablationseffekt-Bewertungselementen zu vergleichen; ein Endgewicht auf Grundlage des Vergleichsergebnisses abzuleiten; und ein Vorhersageergebnis zu erhalten, indem ein vorhergesagter Ablationserfolgsratenwert auf Grundlage des Endgewichts berechnet wird,
wobei die Ablationseffekt-Bewertungselemente eines oder mehrere der folgenden umfassen: ob eine Blutdruck-Basislinie hoch ist, ob der Blutdruck während einer Ablation signifikant angestiegen ist, eine Anzahl von Stellen, an denen die Ablation einen Anstieg des Blutdrucks verursacht hat, und ob vor der Ablation ein Vasodilatator eingenommen wurde, und
wobei der vorhergesagte Ablationserfolgswert nach der Formel M = N + qN berechnet wird, wobei M den vorhergesagten Ablationserfolgsratenwert repräsentiert; N eine voreingestellte Basis-Ablationserfolgsrate repräsentiert; und q das Endgewicht repräsentiert.

2. Die Nervendetektionsvorrichtung nach Anspruch 1, ferner umfassend ein Mehrparameter-Detektionsmodul (70), das elektrisch mit dem Berechnungs- und Steuermodul (20) verbunden ist, wobei das Mehrparameter-Detektionsmodul (70) konfiguriert ist, eines oder mehrere von: einer Temperatur, einer Leistung und einer Zeitdauer während eines Ablationsprozesses zu detektieren,
wobei das Rechen- und Steuermodul (20) ferner dazu konfiguriert ist, eine Ausgangsenergie des Energieerzeugungsmoduls (30) automatisch auf der Grundlage des Detektionsergebnisses des Mehrparameter-Detektionsmoduls (70) anzupassen.

3. Die Nervendetektionsvorrichtung nach Anspruch 1, wobei, wenn das Berechnungs- und Steuermodul (20) bestimmt, dass ein Ablationsziel an der vorgegebenen Stelle vorhanden ist, das Berechnungs- und Steuermodul (20) eine Anzeige ausgibt, das Energieerzeugungsmodul (30) zu steuern, um die vorgegebene Stelle mit einer zweiten Energie zu ablatieren, die durch den Detektionskatheter (50) angewendet wird, wobei die zweite Energie höher als die erste Energie ist, und wobei, wenn das Berechnungs- und Steuermodul (20) bestimmt, dass kein Ablationsziel an der vorgegebenen Stelle vorhanden ist, das Berechnungs- und Steuermodul (20) eine Anzeige zum Fortsetzen oder Stoppen der Detektion ausgibt.

4. Die Nervendetektionsvorrichtung nach Anspruch 3, ferner umfassend ein Anzeigemodul (80) zum Anzeigen eines Bildes der vorgegebenen Stelle, wobei das Anzeigemodul (80) kommunikativ mit dem Berechnungs- und Steuermodul (20) verbunden ist und dazu konfiguriert ist, die Anzeige darzustellen, wenn das Berechnungs- und Steuermodul (20) bestimmt, ob das Ablationsziel an der vorgegebenen Stelle auf der Grundlage des Blutdrucküberwachungsergebnisses des Blutdrucküberwachungsmoduls (40) vorhanden ist, oder wobei das Anzeigemodul (80) kommunikativ mit dem Ablationseffekt-Vorhersagemodul verbunden ist und dazu konfiguriert ist, ein Vorhersageergebnis darzustellen.

5. Die Nervendetektionsvorrichtung nach Anspruch 4, wobei die Anzeige mit einem Text und einem Farbblock wiedergegeben wird, in der Weise dargestellt, dass: ein mit dem Text "Sehr empfehlenswert" oder "Empfohlen" assoziierter Farbblock in einer vorbestimmten ersten Farbe oder einer vorbestimmten zweiten Farbe angezeigt wird, wenn das Blutdruckvariationsmuster des Patienten mit einem vordefinierten ersten Blutdruckvariationsmuster kompatibel ist; oder ein mit dem Text "Nicht Empfohlen" assoziierter Farbblock in einer vorbestimmten dritten Farbe angezeigt wird, wenn das Blutdruckvariationsmuster des Patienten mit einem vordefinierten zweiten Blutdruckvariationsmuster kompatibel ist.

6. Die Nervendetektionsvorrichtung nach Anspruch 3, wobei das Energieerzeugungsmodul (30) ein Hochfrequenz-,RF-, Energie-Modul umfasst, das dazu konfiguriert ist, die erste Energie bereitzustellen, die eine Ablationsenergie mit niedriger Leistung ist, und die zweite Energie bereitzustellen, die eine Ablationsenergie mit hoher Leistung ist.

7. Die Nervendetektionsvorrichtung nach Anspruch 3, wobei das Energieerzeugungsmodul (30) ein Stimulationsmodul umfasst, das dazu konfiguriert ist, die erste Energie bereitzustellen, die eine Stimulationsenergie ist, und ein RF-Energie-Modul umfasst, das dazu konfiguriert ist, die zweite Energie bereitzustellen, die eine Ablationsenergie mit hoher Leistung ist.

8. Die Nervendetektionsvorrichtung nach Anspruch 1, wobei das Energieerzeugungsmodul (30) eines oder mehrere von: einem RF-Energie-Modul, einem Pulsenergie-Modul, einem Lasermodul, einem Ultraschallmodul, einem Strahlungsmodul, einem optischen Energiemodul und einem Kryoenergie-Modul umfasst und dazu konfiguriert ist, eines oder mehrere von: einer RF-Energie, einer Pulsenergie, einer Laserenergie, einer Ultraschallenergie, einer Strahlungsenergie, einer optischen Energie und einer Kryoenergie bereitzustellen.

9. Die Nervendetektionsvorrichtung nach Anspruch 1, ferner umfassend ein Stromversorgungsmodul (10), das konfiguriert ist, jedes des Berechnungs- und Steuermoduls (20), des Energieerzeugungsmoduls (30) und des Blutdrucküberwachungsmoduls (40) mit Strom zu versorgen.

10. Die Nervendetektionsvorrichtung nach Anspruch 1, wobei es sich bei der Arterie um eine Nierenarterie oder eine Aorta handelt.

11. Ein computerlesbares Speichermedium mit einem darauf gespeicherten Computerprogramm, das, sobald es durch einen Prozessor der Nervendetektionsvorrichtung nach Anspruch 1 ausgeführt wird, ein Ablationseffekt-Vorhersageverfahren implementiert, umfassend: Erwerben von Bewertungskriterien, die mit einer Vielzahl von Ablationseffekt-Bewertungselementen assoziiert sind; Vergleichen der Bewertungskriterien mit jeweiligen Ablationseffekt-Bewertungselementen; Ableiten eines Endgewichts auf der Grundlage des Vergleichsergebnisses; und Erhalten eines Vorhersageergebnisses durch Berechnen eines vorhergesagten Ablationserfolgsratenwerts auf der Grundlage des Endgewichts, und
Identifizieren, auf der Grundlage des Blutdrucküberwachungsergebnisses, eines Blutdruckvariationsmusters des Patienten während der Detektion einer vorgegebenen Stelle durch ein Energieerzeugungsmodul mit einer ersten Energie, und Bestimmen, dass ein Ablationsziel an der vorgegebenen Stelle vorhanden ist, wenn das Blutdruckvariationsmuster des Patienten mit einem vordefinierten ersten Blutdruckvariationsmuster kompatibel ist, oder Bestimmen, dass kein Ablationsziel an der vorgegebenen Stelle vorhanden ist, wenn das Blutdruckvariationsmuster des Patienten mit einem vordefinierten zweiten Blutdruckvariationsmuster kompatibel ist,
wobei das vordefinierte erste Blutdruckvariationsmuster einen kontinuierlichen Anstieg über einen vorbestimmten Betrag oder einen Abfall, gefolgt von einem Anstieg über eine Blutdruck-Basislinie hinaus, des Blutdrucks des Patienten während eines vorbestimmten Zeitraums nach Beginn der Detektion der vorgegebenen Stelle durch das Energieerzeugungsmodul mit der ersten Energie aufweist,
wobei das vordefinierte zweite Blutdruckvariationsmuster einen Abfall, gefolgt von einem Anstieg unterhalb der Blutdruck-Basislinie, ein Plateau oder einen kontinuierlichen Abfall des Blutdrucks des Patienten während eines vorbestimmten Zeitraums nach Beginn der Detektion der vorgegebenen Stelle durch das Energieerzeugungsmodul (30) mit der ersten Energie aufweist,
wobei die Blutdruck-Basislinie der Blutdruck des Patienten vor der Detektion der vorgegebenen Stelle durch das Energieerzeugungsmodul (30) mit der ersten Energie ist,
wobei die Ablationseffekt-Bewertungselemente eines oder mehrere von: ob eine Blutdruck-Basislinie hoch ist, ob der Blutdruck während einer Ablation signifikant angestiegen ist, eine Anzahl von Stellen, an denen die Ablation einen Anstieg des Blutdrucks verursacht hat, und ob vor der Ablation ein Vasodilatator eingenommen wurde, umfassen, und
wobei der vorhergesagte Ablationserfolgsratenwert gemäß M = N + qN berechnet wird, wobei M den vorhergesagten Ablationserfolgsratenwert darstellt; N eine voreingestellte grundlegende Ablationserfolgsrate darstellt; und q das Endgewicht darstellt.

## Revendications

1. Un dispositif de détection nerveuse, comprenant un module de calcul et de contrôle (20), un module de génération d'énergie (30), un module de surveillance de la pression artérielle (40) et un module de prédiction de l'effet d'ablation prédéfini avec une pluralité d'éléments d'évaluation de l'effet d'ablation et des pondérations prédéfinies associées, le module de calcul et de contrôle (20) étant connecté électriquement à chacun des modules de génération d'énergie (30) et de surveillance de la pression artérielle (40),
(S1) dans lequel le module de génération d'énergie (30) est configuré pour se connecter à un cathéter de détection (50) et pour détecter un site donné dans une artère en fournissant une première énergie au cathéter de détection (50),
(S2) dans lequel le module de surveillance de la pression artérielle (40) est configuré pour se connecter à un capteur de pression artérielle (60), lequel capteur de pression artérielle (60) est configuré pour surveiller la pression artérielle d' un patient avant et pendant la détection pour le site donné par le module de génération d'énergie (30) avec la première énergie, et pour produire un résultat de surveillance de la pression artérielle, et dans lequel le module de surveillance de la pression artérielle (40) est en outre configuré pour transmettre le résultat de surveillance de la pression artérielle au module de calcul et de contrôle (20),
(S3) dans lequel le module de calcul et de contrôle (20) est configuré pour déterminer si une cible d'ablation est présente à l'emplacement donné en fonction du résultat de la surveillance de la pression artérielle et pour identifier, sur la base du résultat de la surveillance de la pression artérielle, le modèle de variation de la pression artérielle d' un patient pendant la détection de l'emplacement donné par le module de génération d'énergie (30) avec la première énergie, et pour déterminer qu'une cible d'ablation est présente à l'emplacement donné si le modèle de variation de la pression artérielle du patient est compatible avec un premier modèle de variation de la pression artérielle prédéfini, ou pour déterminer qu'aucune cible d'ablation n'est présente à l'emplacement donné si le modèle de variation de la pression artérielle du patient est compatible avec un deuxième modèle de variation de la pression artérielle prédéfini,
dans lequel le premier modèle prédéfini de variation de la pression artérielle présente une augmentation continue au-delà d'une quantité prédéterminée, ou une baisse suivie d'une augmentation au-delà d' une valeur de base de la pression artérielle, de la pression artérielle du patient pendant une période de temps prédéterminée après le début de la détection pour le site donné par le module de génération d'énergie (30) avec la première énergie,
dans lequel le deuxième modèle prédéfini de variation de la pression artérielle présente une chute suivie d'une hausse en dessous de la ligne de base de la pression artérielle, un plateau ou une chute continue de la pression artérielle du patient pendant une période de temps prédéterminée après le début de la détection pour le site donné par le module de génération d'énergie (30) avec la première énergie,
dans lequel la pression artérielle de base est la pression artérielle du patient avant la détection pour le site donné par le module de génération d'énergie (30) avec la première énergie,
dans lequel le module de prédiction de l'effet d'ablation est configuré pour : acquérir les critères d'évaluation associés aux éléments d'évaluation de l'effet d'ablation respectifs ; comparer les critères d'évaluation avec les éléments d'évaluation de l'effet d'ablation respectifs ; dériver un poids final basé sur le résultat de la comparaison et obtenir un résultat de prédiction en calculant une valeur de taux de réussite d'ablation prédite basée sur le poids final,
dans lequel les éléments d'évaluation de l'effet de l'ablation comprennent un ou plusieurs des éléments suivants : si la pression artérielle de base est élevée, si la pression artérielle a augmenté de manière significative pendant l'ablation, le nombre de sites où l'ablation a provoqué une augmentation de la pression artérielle, et si un vasodilatateur a été pris avant l'ablation, et
dans lequel la valeur du taux de réussite de l'ablation prédit est calculée selon M=N+qN, où M représente la valeur du taux de réussite de l'ablation prédit; N représente un taux de réussite de l'ablation de base prédéfini; et q représente le poids final.

2. Le dispositif de détection nerveuse selon la revendication 1, comprenant en outre un module de détection multiparamètre (70) connecté électriquement au module de calcul et de contrôle (20), dans lequel le module de détection multiparamètre (70) est configuré pour détecter un ou plusieurs des éléments suivants : une température, une puissance et un temps pendant un processus d'ablation,
dans lequel le module de calcul et de contrôle (20) est en outre configuré pour ajuster automatiquement l'énergie de sortie du module de génération d'énergie (30) en fonction du résultat de détection du module de détection multiparamètre (70).

3. Le dispositif de détection nerveuse selon la revendication 1, dans lequel lorsque le module de calcul et de contrôle (20) détermine qu'une cible d'ablation est présente à l'emplacement donné, le module de calcul et de contrôle (20) émet une indication de contrôle du module de génération d'énergie (30) pour ablater le site donné avec une seconde énergie appliquée par le cathéter de détection (50), où la seconde énergie est supérieure à la première énergie, et où lorsque le module de calcul et de contrôle (20) détermine qu'aucune cible d'ablation n'est présente au site donné, le module de calcul et de contrôle (20) émet une indication de continuer ou d'arrêter la détection.

4. Le dispositif de détection nerveuse selon la revendication 3, comprenant en outre un module d'affichage (80) pour afficher une image du site donné, dans lequel le module d'affichage (80) est connecté de manière communicative au module de calcul et de contrôle (20) et est configuré pour afficher l'indication lorsque le module de calcul et de contrôle (20) détermine si la cible d'ablation est présente au site donné sur la base du résultat de la surveillance de la pression artérielle du module de surveillance de la pression artérielle (40), ou dans lequel le module d'affichage (80) est connecté de manière communicative à un module de prédiction de l'effet d'ablation et est configuré pour afficher un résultat de prédiction.

5. Le dispositif de détection nerveuse selon la revendication 4, dans lequel l'indication est rendue par un texte et un bloc de couleur, présentés de la manière suivante : affichage d'un bloc de couleur associé au texte « Fortement recommandé » ou « Recommandé » dans une première couleur prédéterminée ou une deuxième couleur prédéterminée si le profil de variation de la pression artérielle d' un patient est compatible avec un premier profil de variation de la pression artérielle prédéfini ; ou affichage d'un bloc de couleur associé au texte « Non recommandé » dans une troisième couleur prédéterminée si le profil de variation de la pression artérielle d' un patient est compatible avec un deuxième profil de variation de la pression artérielle prédéfini.

6. Le dispositif de détection nerveuse selon la revendication 3, dans lequel le module de génération d'énergie (30) comprend un module d'énergie radiofréquence (RF) configuré pour fournir la première énergie qui est une énergie d'ablation de faible puissance et la seconde énergie qui est une énergie d'ablation de haute puissance.

7. Le dispositif de détection nerveuse selon la revendication 3, dans lequel le module de génération d'énergie (30) comprend un module de stimulation configuré pour fournir la première énergie qui est une énergie de stimulation, et un module d'énergie RF configuré pour fournir la seconde énergie qui est une énergie d'ablation de haute puissance.

8. Le dispositif de détection nerveuse selon la revendication 1, dans lequel le module de génération d'énergie (30) comprend un ou plusieurs des éléments suivants : un module d'énergie RF, un module d'énergie d'impulsion, un module laser, un module ultrasonique, un module de rayonnement, un module d'énergie optique et un module d'énergie cryogénique, et est configuré pour fournir une ou plusieurs des énergies suivantes : une énergie RF, une énergie d'impulsion, une énergie laser, une énergie ultrasonique, une énergie de rayonnement, une énergie optique et une énergie cryogénique.

9. Le dispositif de détection nerveuse selon la revendication 1, comprenant en outre un module d'alimentation (10) configuré pour alimenter chacun des modules de calcul et de contrôle (20), de génération d'énergie (30) et de surveillance de la pression artérielle (40).

10. Le dispositif de détection nerveuse selon la revendication 1, dans lequel l'artère est une artère rénale ou une aorte.

11. Un support de stockage lisible par ordinateur sur lequel est stocké un programme informatique, qui, une fois exécuté par un processeur du dispositif de détection nerveuse selon la revendication 1, met en œuvre une méthode de prédiction de l'effet d'ablation comprenant : l'acquisition de critères d'évaluation associés à une pluralité d'éléments d'évaluation de l'effet d'ablation ; la comparaison des critères d'évaluation avec les éléments d'évaluation de l'effet d'ablation respectifs ; la dérivation d'une pondération finale basée sur le résultat de la comparaison ; et l'obtention d'un résultat de prédiction en calculant une valeur de taux de réussite d'ablation prédite basée sur la pondération finale,
identifier, à partir du résultat de la surveillance de la pression artérielle, le profil de variation de la pression artérielle d' un patient lors de la détection d'une cible d'ablation à un site donné par un module de génération d'énergie avec une première énergie, et déterminer qu'une cible d'ablation est présente au site donné si le profil de variation de la pression artérielle du patient est compatible avec un premier profil de variation de la pression artérielle prédéfini, ou déterminer qu'aucune cible d'ablation n'est présente au site donné si le profil de variation de la pression artérielle du patient est compatible avec un deuxième profil de variation de la pression artérielle prédéfini,
dans lequel le premier modèle prédéfini de variation de la pression artérielle présente une augmentation continue au-delà d'une valeur prédéterminée, ou une baisse suivie d'une augmentation au-delà d'une valeur de base de la pression artérielle, de la pression artérielle du patient pendant une période de temps prédéterminée après le début de la détection pour le site donné par le module de génération d'énergie avec la première énergie,
dans lequel le deuxième modèle prédéfini de variation de la pression artérielle présente une chute suivie d'une hausse en dessous de la ligne de base de la pression artérielle, un plateau ou une chute continue de la pression artérielle du patient pendant une période de temps prédéterminée après le début de la détection pour le site donné par le module de génération d'énergie (30) avec la première énergie,
dans lequel la pression artérielle de base est la pression artérielle du patient avant la détection pour le site donné par le module de génération d'énergie (30) avec la première énergie,
dans lequel les éléments d'évaluation de l'effet de l'ablation comprennent un ou plusieurs des éléments suivants : si la pression artérielle de base est élevée, si la pression artérielle a augmenté de manière significative pendant l'ablation, le nombre de sites où l'ablation a provoqué une augmentation de la pression artérielle et si un vasodilatateur a été pris avant l'ablation, et dans lequel la valeur du taux de réussite de l'ablation prédit est calculée selon M = N+ qN, où M représente la valeur du taux de réussite de l'ablation prédit ; N représente un taux de réussite de l'ablation de base prédéfini ; et q représente le poids final.
